# EUROPEAN PATENT SPECIFICATION

(11) **EP 0 222 385 B1**
(45) Date of publication and mention of the grant of the patent: **03.02.1993**
(21) Application number: 86115706.3
(22) Date of filing: 12.11.1986
(51) Int. Cl.: A61K 31/565, A61K 31/57, A61K 31/05

(54) **Sex hormones for the treatment of immunodeficiency diseases**
Geschlechtshormone zur Behandlung von Immun-Mangel-Krankheiten
Hormones sexuelles pour le traitement des maladies de l'immunodéficience

(30) Priority: 13.11.1985 JP 255791/85
(43) Date of publication of application: 20.05.1987
(73) Proprietor: Research Development Corporation of Japan, Chiyoda-ku Tokyo (JP); Ueno Seiyaku Kabushikikaisha, Osaka-shi Osaka-fu (JP)
(72) Inventor: Kuno, Sachiko, Ibaraki-shi, Osaka-fu (JP); Ueno, Ryuji, Sakyo-ku Kyoto-shi Kyoto-fu (JP); Hayaishi, Osamu Royal Court Shimogamo 205, Sakyo-ku Kyoto-shi Kyoto-fu (JP)
(74) Representative: VOSSIUS & PARTNER

(56) References cited:
- EP-A- 0 159 739
- DE-A- 3 812 595
- SCIENCE, vol. 227, no. 4684, January 18, 1985, pages 257-261, C.J. GROSSMAN: "Interaction between the gonadal steroids and immune system"
- CONTRACEPCION, vol. 28, no. 6, December 1983, pages 587-601; H. KUHL et al.: "The effect of sex steroids and hormonal contraceptives upon thymus and spleen of intact female rats"
- INTERNATIONAL JOURNAL OF CANCER, vol. 37, no. 1, January 15, 1986, pages 67-72, Alan R. Liss. Inc.; P.D. MARKHAM et al.: "Hydrocortisone and some other hormones enhance the expression of HTLV-III"
- IMMUNOLOGY, vol. 39, no. 1, 1980, pages 37-46, Blackwell Scientific Publications; Y. HIROTA et al.: "The B-cell development independent of the bursa of Fabricius but dependent upon the thymus in chickens treated with testosterone propionate"
- Arzneimitt. Forsch. 15, p. 666-670
- Br. J. Pharmac. Chemother. 32, p. 253-261

## Description

The present invention relates to the use of sex hormones for the manufacture of a medicament for the treatment of immunodeficiency diseases associated with the lowering of the cellular immunological competence.

Immunodeficiency diseases, including AIDS (acquired immunological deficiency syndrome), are attracting many people's interest due to their unfavorable prognosis. AIDS is known to occur frequently in homosexuals, being characterized by the clinical conditions such as pneumonia, sarcoma, etc., and to cause a high rate of death of more than 70 % because of the destruction of the immune response. It is also known that the helper T cells are specifically put into disorder.

As a result of the search for the causes of the frequent occurrences of AIDS among the homosexuals, the present inventors have found out the following facts:
(1) As shown in the following Table 1, human semen contains a large amount of prostaglandin E₂,
(2) In vivo animal experiments reveal that prostaglandin E₂ lowers the cellular immunological competence of male lymphocytes, but not the cellular immunological competence of female lymphocytes.
(3) The reason why the homosexual male can be affected by pathogens such as the AIDS virus is that prostaglandin E₂ which is contained in a large amount in semen is injected into the rectum by the homosexual act and absorbed in the body thereby causing the lowering of the cellular immunological competence.

They have further found out:
(4) that prostaglandin D₂, A₂ and J₂ cause the lowering of the cellular immunological competence in both males and females, and
(5) that the lowering of the immunological competence caused by prostaglandins in both males and females can be counteracted by the administration of male or female steroid hormones.

The present invention has been made on the basis of the above findings.

**Table 1**

| Concentration of prostaglandins in human semen | | |
|---|---|---|
| | 1st time (µg/ml) | 2nd time (µg/ml) |
| PGE₂ | 66.7 | 78.1 |
| PGF_{2α} | 7.8 | 9.1 |
| PGD₂ | 0.17 | 0.19 |
| (NOTE): Determined by radioimmunoassay. Samples were collected from two men. | | |

A comprehensive review on sex hormones can be found in Kirk-Othmer Encyclopedia of Chemical Technology, 3rd Edition, Volume 12, Pages 618-657 (John Wiley and Sons, New York, U.S.A., 1980). EP-A- 159 739 discloses Δ ⁴ and 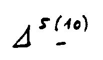
oestrene derivatives with an alkyl substituent in position 6 or 7, which possess immunomodulating properties.

One aspect of the present invention is the use of a sex hormone selected from testosterone, testosterone propionate, methyltestosterone, androsterone, progesterone, mestanolone, methenolone enanthate, androstanolone, methandienone, oxandrolone, fluoxymesterone, stanozolol, thiomesterone, cyproterone acetate, estradiol, ethynyl estradiol, estradiol benzoate, estradiol cypionate, and estriol for the manufacture of a medicament for treatment of immunodeficiency diseases associated with the lowering of the cellular immunological competence.

The term treatment herein is intended to cover all controls including prevention and therapy.

The immunodeficiency diseases to be treated include the lowering of the cellular immunological competence, especially the lowering of the immunological competence of T-cells,e.g., helper T-cells. Typical ones are the lowering of the immunological competence induced by prostaglandins and the lowering of the immunological competence seen in homosexual males.

As described above, the sex hormones improve the lowered immunological competence. This action is seen with both the male hormones and the female hormones, but is stronger in the case of the female hormones. Due to their action, the sex hormones are effective at the recovery of the immunological competence in patients who show a lowering of the immunological competence and at the prevention of the lowering of the immunological competence in patients who are for example treated with prostaglandin. Especially, the sex hormones are effective at the improvement of the immune-dysfunction of homosexual males. Further, they are effective at the treatment of diseases such as ARC or AIDS caused by the lowering of immunological competence or accompanied by the lowering of immunological competence.

The amount of the sex hormones to be used for the above manufacture is approximately the same as that used generally in the manufacture of medicaments used for sex hormone therapy. For example,in the case of a human being, the amount may be 0.01 - 20 mg (preferably 0.05 - 5 mg) (in case of estrogens)or 0.5 - 50 mg (preferably 1 - 10 mg) (in case of gestagens)or 5 - 100 mg (preferably 10 - 50 mg) (in case of androgens), administered daily 1 - 4 times, or in the form of a preparation having sustained effect. The medicaments can be manufactured for any administration, e.g. peroral, intrarectal, or by injection, but preferably for peroral administration or intramuscular injection.

The manufactured medicaments may contain the sex hormone as an active ingredient in the form of any ordinary pharmaceutical preparation mixed with pharmaceutical carriers such as organic or inorganic solid or liquid vehicles suitable for the administration method, e.g. peroral, intrarectal and by injection. Such preparations include solid state tablets, granules, dusts, capsules, and liquid state solutions, suspensions and emulsifiers. As the carriers, there may be used starch, lactose, glucose, sucrose, dextrin, cellulose, paraffin, fatty acid glyceride, water and alcohols. Adjuvant, stabilizer, humidifier, emulsifier, lubricant, binder, and other conventional additives may be added if necessary.

The present invention is illustrated in more detail by way of the examples and its effect demonstrated by way of the test examples.

### Example 1

| | |
|---|---|
| Estradiol | 10 mg |
| Vegetable oil | q.s. to 10 ml |

### Example 2

| | |
|---|---|
| Estradiol | 100 mg |

An aqueous suspension (10 ml) is prepared by an ordinary procedure.

### Example 3

| | |
|---|---|
| Testosterone propionate | 250 mg |
| Vegetable oil | q.s. to 10 ml |

### Example 4

| | |
|---|---|
| Methyl testosterone | 10 mg |
| Lactose | 48 mg |
| Microcrystalline cellulose | 35 mg |
| Corn starch | 5 mg |
| Hydroxypropyl cellulose | 1 mg |
| Magnesium stearate | 1 mg |

The above compounds are mixed and pressed according to usual procedures to make a tablet.

### Example 5

| | |
|---|---|
| Methandrostelone | 20 mg |
| Lactose | 180 mg |

The above compounds are mixed and filled in a capsule.

### Example 6

| | |
|---|---|
| Progesterone | 100 mg |
| Vegetable oil | q.s. to 10 ml |

### Test Example 1 (Cellular immunity of lymphocytes)

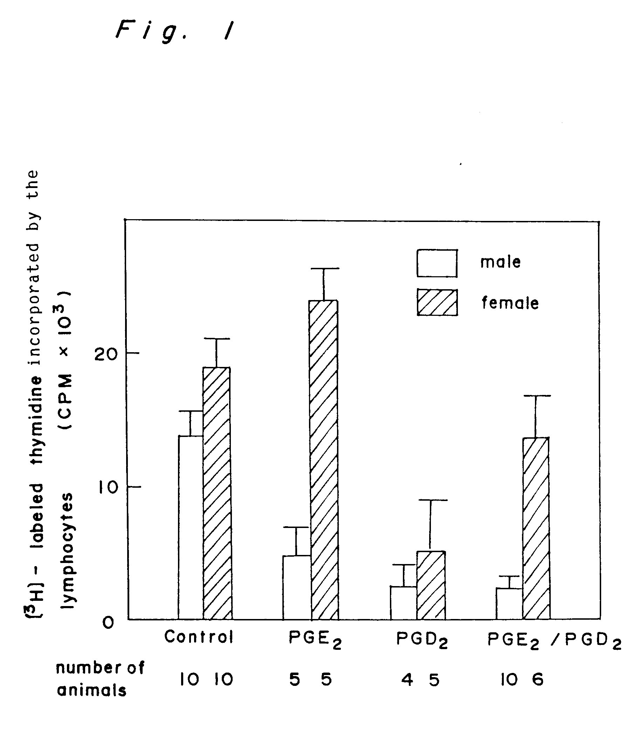

Prostaglandin (PG)E₂ and/or D₂ (0.5 mg/kg) were intrarectally administered to rats (Wistar strain, SPF, 8 weeks old, male and female) once a day for 7 days. Three hours after the final administration, blood was collected and the lymphocytes separated. The blastogenesis of T lymphocytes against PHA (phytohemagglutinin) was investigated by measuring the incorporation of labeled thymidine. The results are shown in Fig. 1.

It can be observed from Fig. 1 that in the case of intrarectally administered prostaglandin E₂ the immunological competence is lowered in males only, while in the case of intrarectally administered prostaglandin D₂ the immunological competences are lowered in both males and females. Human semen contains a large amount of prostaglandin E derivatives (Table I). Therefore, the lowering of immunological competence in homosexual males may be due to the absorption of seminal prostaglandins through sexual activities.

### Test Example 2 (Effect of sex hormones)

Prostaglandin E₂ was intrarectally administered to male rats (Wistar strain, 3 weeks old, male, n = 3) in an amount of 0.5 mg/kg once a day for 7 days. Prior to it, they were subcutaneously injected for 7 days with 1 mg/day of testosterone (TES) or estradiol (EST) suspended in olive oil.

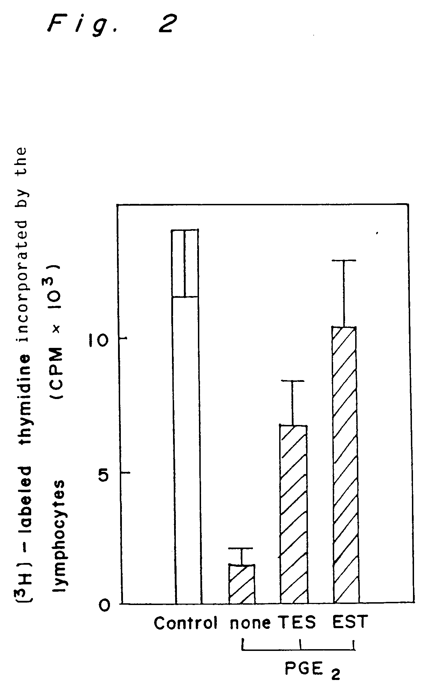

Three hours after the final administration, blastogenesis of lymphocytes was determined in the same manner as described in Test Example 1. The results are shown in Fig. 2.

From Fig. 2 it can be seen that testosterone and estradiol inhibit the lowering of the cellular immunity, and that estradiol has a stronger inhibitory action than testosterone.

### Test Example 3 (Effect of sex hormones)

Prostaglandins D₂, A₂, and J₂ were intrarectally administered to male rats (Wistar strain, 3 weeks old, male, n = 2) in an amount of 0.5 mg/kg, once a day for 7 days. Prior to prostaglandin administration, they were subcutaneously injected for 7 days with 1 mg/day testosterone (TES) or estradiol (EST) suspended in olive oil. The blastogenesis of lymphocytes was determined as previously mentioned.

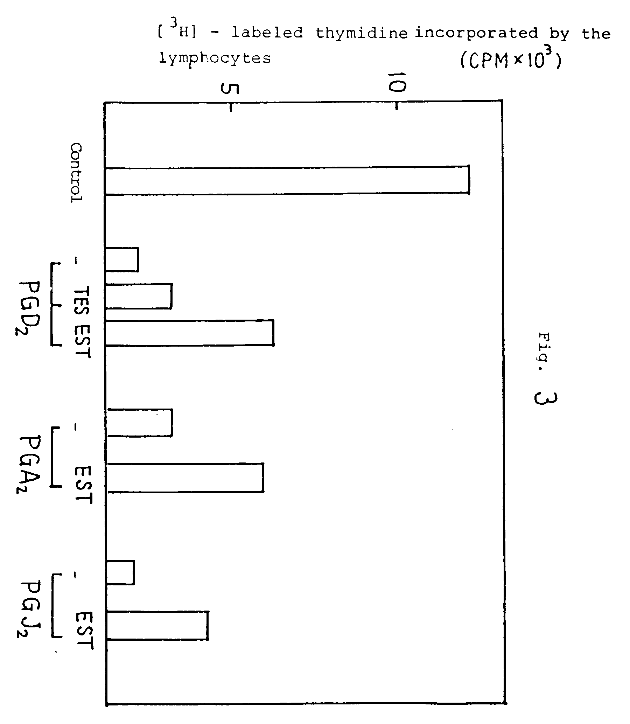

Fig. 3 shows that prostaglandin D₂, A₂ and J₂ lower T lymphocytes blastogenic response. Further, the lowering of cellular immunity caused by prostaglandins such as prostaglandin D₂, A₂ and J₂ is also blocked by the treatement with either testosterone or estradiol, which is shown in Fig. 3. Therefore, sex steroids are expected to express an anti-immunosuppressive effect against all the immunosuppressive prostaglandins.

## Claims

1. The use of sex hormones selected from testosterone, testosterone propionate, methyltestosterone, androsterone, progesterone, mestanolone, methenolone enanthate, androstanolone, methandienone, oxandrolone, fluoxymesterone, stanozolol, thiomesterone, cyproterone acetate, estradiol, ethynyl estradiol, estradiol benzoate, estradiol cypionate, and estriol for the manufacture of a medicament for the treatment of immunodeficiency diseases associated with the lowering of the cellular immunological competence.

2. The use according to claim 1, wherein said cellular immunological competence is that of lymphocytes

3. The use according to claim 1, wherein said treatment is aimed at recovering the immunological competence in a patient who shows a lowering of the immunological competence or at preventing the lowering of the immunological competence.

4. The use according to claim 1, wherein said treatment consists in the prevention or therapy of ARC and AIDS.

5. The use according to claim 2, wherein said lymphocytes are T-cells.

6. The use according to claim 1, wherein said sex hormone is testosterone or estradiol.

## Patentansprüche

1. Verwendung von Geschlechtshormonen, ausgewählt aus Testosteron, Testosteron-propionat, Methyltestosteron, Androsteron, Progesteron, Mestanolon, Methenolonenanthat, Androstanolon, Methandienon, Oxandrolon, Fluoxmesteron, Stanozolol, Thiomesteron, Cyproteronacetat, Östradiol, Ethinyl-östradiol, Östradiol-benzoat, Östradiol-cypionat und Östriol zur Herstellung eines Arzneimittels für die Behandlung von Immundefizienz-Erkrankungen, die mit einer Erniedrigung der zellulären immunologischen Kompetenz verbunden sind.

2. Verwendung nach Anspruch 1, wobei die zelluläre immunologische Kompetenz diejenige von Lymphocyten ist.

3. Verwendung nach Anspruch 1, wobei die Behandlung auf die Wiedergewinnung der immunologischen Kompetenz in einem Patienten, der eine Erniedrigung der immunologischen Kompetenz zeigt, oder auf die Verhinderung der Erniedrigung der immunologischen Kompetenz gerichtet ist.

4. Verwendung nach Anspruch 1, wobei die Behandlung in der Prävention oder Therapie von ARC und AIDS besteht.

5. Verwendung nach Anspruch 2, wobei die Lymphocyten T-Zellen sind.

6. Verwendung nach Anspruch 1, wobei das Geschlechtshormon Testosteron oder Östradiol ist.

## Revendications

1. Utilisation d'hormones sexuelles choisies parmi la testostérone, le propionate de testostérone, la méthyltestostérone, l'androstérone, la progestérone, la mestanolone, l'énanthate de méthénolone, l'androstanolone, la méthandiénone, l'oxandrolone, la fluoxymestérone, le stanozolol, la thiomestérone, l'acétate de cyprotérone, l'estradiol, l'éthynylestradiol, le benzoate d'estradiol, le cypionate d'estradiol et l'estriol, pour la préparation d'un médicament pour le traitement des maladies immunodéficitaires associées à une diminution de la compétence immunologique cellulaire.

2. Utilisation selon la revendication 1, dans laquelle ladite compétence immunologique cellulaire est celle des lymphocytes.

3. Utilisation selon la revendication 1, dans laquelle ledit traitement vise à rétablir la compétence immunologique chez un patient qui présente un abaissement de la compétence immunologique ou pour prévenir la diminution de la compétence immunologique.

4. Utilisation selon la revendication 1, dans laquelle ledit traitement consiste en la prévention ou le traitement du complexe lié au SIDA et du SIDA.

5. Utilisation selon la revendication 2, dans laquelle lesdits lymphocytes sont des cellules T.

6. Utilisation selon la revendication 1, dans laquelle ladite hormone sexuelle est la testostérone ou l'estradiol.
